# EUROPEAN PATENT APPLICATION

(11) **EP 4 729 108 A1**
(43) Date of publication of application: **22.04.2026**
(21) Application number: 25205486.1
(22) Date of filing: 29.09.2025
(51) Int. Cl.: A61M 25/10, A61N 5/10, A61M 25/00, A61M 25/01

(54) **BALLOON CATHETER**

(30) Priority: 21.10.2024 KR 20240143849; 18.08.2025 KR 20250113997
(71) Applicant: RADEXEL INC., Chuncheon-si, Gangwon-do 24437 (KR)
(72) Inventor: JUNG, Nuri Hyun, 24363 Chuncheon-si (KR); BAHNG, Jungbae, 30147 Sejong-si (KR); KIM, Seongjun, 04759 Seoul (KR); KIM, Tae Hoon, 03469 Seoul (KR)
(74) Representative: HGF

(57) **Abstract**

The present disclosure relates to a balloon catheter including an external catheter (100); a balloon (200) connected to one end of the external catheter, inflatable and deflatable, and configured to be inserted into or expelled from a target site of a living body; and an internal catheter (300) movable along the external catheter in a first direction for insertion into the balloon or a second direction for expelling the balloon, wherein the internal catheter is expelled from the balloon while the balloon is inflated and fixed to the target site of the living body.

## Description

### [TECHNICAL FIELD]

The present disclosure relates to a balloon catheter.

### [BACKGROUND ART]

Radiation therapy is to irradiate a lesion of a living body with radiation such as X-rays, gamma rays, electron rays, and proton rays to delay or destroy the growth of the lesion tissue of the living body. Here, the lesion tissue may be cancer, and the like.

During radiation therapy, the distribution of the radiation dose may change depending on the density of the medium located in the path through which the radiation beam penetrates the living body. Specifically, if two media with different densities exist in the path through which the radiation penetrates the living body, the distribution of the radiation dose in the boundary area of the two media may be greater than in other areas.

During radiation therapy, to reduce the radiation effect on the surrounding tissue of the lesion tissue of the living body, a balloon may be inserted into the body cavity (hereinafter referred to as the "target site") around the lesion tissue of the living body to fix the lesion tissue and the target site of the living body. Here, the balloon may be inserted into the target site of the living body through an internal catheter (or guide wire). Specifically, for example, the internal catheter may be inserted into the inside of the balloon, and then the balloon and the internal catheter may be inserted into the target site of the living body. At this time, the internal catheter inside the balloon caused the following problems.

First, the internal catheter inside the balloon made it impossible to lower the internal density of the balloon below a certain level. For reference, increasing the density inside the balloon is easy to achieve by simply injecting a high-density material into the balloon.

In addition, the internal catheter inside the balloon causes interference with particle beams during radiation therapy using particles such as electrons, protons, and carbon ions, which changes the point of arrival of the particle beams.

Furthermore, the internal catheter inside the balloon changes its position inside the balloon, causing uncertainty in the radiation dose distribution inside and around the balloon.

In addition, the internal catheter inside the balloon causes secondary electrons to be generated inside the balloon when performing MCRT (Magnetic Controlled Radiation Therapy) that controls the path of radiation using a magnetic field during radiation therapy such as X-rays, reducing the effect of radiation dose control.

Therefore, it is necessary to eject the internal catheter inside the balloon while the balloon is inserted into the target area of the living body. In this case, the general method of ejecting the internal catheter is to eject the internal catheter from inside the balloon while the balloon is not inflated. However, this method has the following problems.

First, there is a problem that the position of the balloon changes due to various directional forces from the internal organs and muscles inside the living body at the moment the internal catheter is ejected from inside the balloon while the balloon is inserted into the target area of the living body.

In addition, when there is pressure inside the living body, there is a problem that the balloon get stuck in the internal catheter at the moment the internal catheter is ejected from inside the balloon while the balloon is inserted into the target area of the living body.

It is important for radiation therapy to maintain the position of the balloon at the target area of the living body. Therefore, the conventional method of ejecting the internal catheter from inside the balloon while the balloon is not inflated is difficult to apply due to changes in the position of the balloon, and as a result, the balloon capable of ejecting the internal catheter has not been used during radiation therapy.

### [Prior art document]

(Patent Document 1) Korea Patent Registration No. 10-2013-0009445 (Published on January 23, 2013)

### [DETAILED DESCRIPTION OF THE INVENTION]

### [TECHINICAL PROBLEM]

The present disclosure is to provide a balloon catheter that can improve the accuracy of radiation therapy for lesion tissue and target area of a living body by allowing an internal catheter inserted into the balloon to be discharged while the balloon is inflated and fixes the lesion tissue and target area of the living body.

The problems to be solved by the present disclosure are not limited to the problems mentioned above, and other problems that are not mentioned will be clearly understood by those skilled in the art from the description below.

### [TECHNICAL SOLUTION]

According to an embodiment of the present disclosure, a balloon catheter includes an external catheter; a balloon connected to one end of the external catheter, inflatable and deflatable, and configured to be inserted into or expelled from a target site of a living body; and an internal catheter movable along the external catheter in a first direction for insertion into the balloon or a second direction for expelling the balloon, wherein the internal catheter is expelled from the balloon while the balloon is inflated and fixed to the target site of the living body.

Furthermore, the balloon catheter may further include a first stopper that limits a range of movement of the internal catheter in the second direction to prevent the internal catheter from being dislodged from the external catheter.

Furthermore, the first stopper may include a hook groove formed in the external catheter; and a hook formed in the internal catheter and engaging the hook groove as the internal catheter moves in the second direction.

Furthermore, the first stopper may include a plug connected to one side of the internal catheter, and the plug may include: a plug body connected to one side of the internal catheter; a channel formed in the plug body and communicating with the internal catheter; and a step protruding from one side of the plug body and engaging a sealing part of the external catheter as the internal catheter moves in the second direction.

Furthermore, the plug may further include a catch protrusion from the other side of the plug body and catching and fixed on an inner circumference of the internal catheter.

Furthermore, the balloon catheter may further include a second stopper configured to limit a movement range of the internal catheter in the first direction and a movement range of the second direction to prevent the internal catheter from dislodging from the external catheter.

Furthermore, the second stopper may include two catch protrusions spaced apart with a spacing and connected along a movement direction of the internal catheter to the outer circumference of the external catheter; and a moving protrusion connected to the outer circumference of the internal catheter and is arranged between the two catch protrusions.

Furthermore, the balloon catheter may further include a driving part that moves the internal catheter in the second direction while the balloon is inflated and fixed to the target site of the living body.

Furthermore, the driving part may include an actuator configured to move the internal catheter in the second direction.

Furthermore, the driving part may include a fluid supply part configured to inject fluid into the balloon so that pressure from the fluid injected into the balloon assists the movement of the internal catheter in the second direction.

Furthermore, the external catheter may include: a guide part connected to the ballon to guide the movement of the internal catheter; and a sealing part protruding from the guide part toward the internal catheter and in close contact with the internal catheter.

Furthermore, the balloon catheter may further include a wing part protruding from an outer periphery of the external catheter to limit a depth at which the external catheter is inserted into the target site of the living body.

Other specific details of the present disclosure are included in the detailed description and drawings.

### [ADVANTAGEOUS EFFECTS OF THE INVENTION]

According to one embodiment of the present disclosure, the balloon catheter can discharge the internal catheter inserted inside the balloon while the balloon is inflated and fixes lesion tissue and target area of a living body, thereby improving the accuracy of radiation therapy for the lesion tissue and target area of the living body during radiation therapy.

The effects of the present disclosure are not limited to the effects mentioned above, and other effects not mentioned will be clearly understood by those skilled in the art from the description below.

### [DESCRIPTION OF THE DRAWINGS]

FIG. 1 is a cross-sectional view illustrating a balloon catheter according to one embodiment of the present disclosure.
FIG. 2 is a cross-sectional view illustrating the balloon of FIG. 1 in an inflated state.
FIG. 3 is a cross-sectional view illustrating a first stopper of a balloon catheter according to an embodiment of the present disclosure.
FIG. 4 is a cross-sectional view illustrating the balloon of FIG. 3 in an inflated state.
FIG. 5 is a cross-sectional view illustrating a second stopper of a balloon catheter according to one embodiment of the present disclosure.
FIG. 6 is a cross-sectional view illustrating the balloon of FIG. 5 in an inflated state.
FIGS. 7 to 10 are cross-sectional views illustrating an operation process of a balloon catheter according to another embodiment of the present disclosure.
FIG. 11 is a schematic diagram illustrating a state in which a balloon of a balloon catheter according to one embodiment of the present disclosure is fixed to a target site of a living body.
FIG. 12 is a schematic diagram illustrating a state in which a balloon of a conventional balloon catheter is fixed to a target site of a living body.
FIG. 13 is a cross-sectional diagram illustrating a process of radiation therapy of a target site of a living body using a balloon catheter according to one embodiment of the present disclosure.
FIG. 14 is a cross-sectional diagram illustrating a process of radiation therapy of a target site of a living body using a conventional balloon catheter.
FIG. 15 is a cross-sectional view illustrating an MCRT treatment process of a target area of a living body using a balloon catheter according to one embodiment of the present disclosure.
FIG. 16 is a cross-sectional view illustrating an MCRT treatment process of a target area of a living body using a balloon catheter according to one embodiment of the present disclosure.
FIG. 17 is a perspective view of a balloon catheter according to still another embodiment of the present disclosure, including a plug and wing part.
FIG. 18 is a cross-sectional view of a balloon catheter according to still another embodiment of the present disclosure, including a plug and wing part.
FIG. 19 is a cross-sectional view showing an external catheter of a balloon catheter according to still another embodiment of the present disclosure.
FIG. 20 is a perspective view showing a plug of a balloon catheter according to still another embodiment of the present disclosure.
FIG. 21 is an exploded cross-sectional view illustrating a plug and an internal catheter of a balloon catheter according to still another embodiment of the present disclosure.
FIGS. 22 and 23 are cross-sectional views illustrating the operation of a balloon catheter according to still another embodiment of the present disclosure.

### [DETAILED DESCRIPTION]

The advantages and features of the present disclosure, and methods for achieving them, will become clear with reference to the embodiments described in detail below along with the accompanying drawings. However, the present disclosure is not limited to the embodiments disclosed below and may be implemented in various different forms. The present embodiments are merely provided to ensure that the present disclosure is complete, and provided to fully convey the scope of the present disclosure to those skilled in the art to which the present disclosure pertains. The present disclosure is defined only by the scope of the claims.

The terminology used herein is for the purpose of describing embodiments and is not intended to limit the disclosure. As used herein, singular forms also include plural forms, unless specifically stated otherwise in the context. As used in the specification, "comprises" and/or "comprising" does not exclude the presence or addition of one or more other elements in addition to the mentioned elements. The same reference numerals refer to the same elements throughout the specification, and "and/or" includes each and every combination of one or more of the referenced elements. Although "first", "second", and the like are used to describe various components, these components are of course not limited by these terms. These terms are merely used to distinguish one component from another. Therefore, the first component mentioned below may also be the second component within the technical spirit of the present disclosure.

Unless otherwise defined, all terms (including technical and scientific terms) used in this specification may be used with meanings commonly understood by those skilled in the art to which this disclosure pertains. Additionally, terms defined in commonly used dictionaries are not to be interpreted ideally or excessively unless clearly specifically defined.

Hereinafter, the embodiments of the present disclosure will be described in detail with reference to the attached drawings.

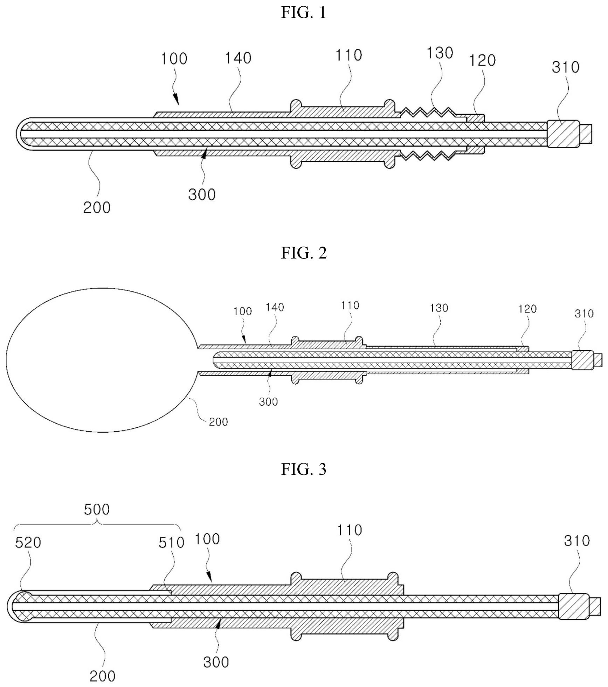

FIG. 1 is a cross-sectional view illustrating a balloon catheter according to one embodiment of the present disclosure, and FIG. 2 is a cross-sectional view illustrating the balloon of FIG. 1 in an inflated state.

As shown in FIG. 1, the balloon catheter according to one embodiment of the present disclosure may include an external catheter 100, a balloon 200, an internal catheter 300, and a driving part.

The external catheter 100 may serve as a basic body of the present disclosure. The external catheter 100 may be open on both sides. Here, one side of the external catheter 100 may be connected by the balloon 200. In addition, the interior of the external catheter 100 may be penetrated by the internal catheter 300. Furthermore, a handle 110 for grasping the external catheter 100 may be formed on the other side of the external catheter 100. Here, the center of the handle 110 may have a smaller outer diameter than the two sides of the handle 110, in other words, the two sides of the handle 110 may have a multi-stage shape that protrudes from the center of the handle 110. Accordingly, the two sides of the handle 110 may serve to prevent the operator's hand grasped at the center of the handle 110 from being separated in the longitudinal direction of the handle 110.

As an example, the outer diameter of the external catheter 100 may be 10 mm to 30 mm, but the present disclosure is not limited thereto. As another example, the length of the external catheter 100 may be 50 mm to 1,000 mm, but the present disclosure is not limited thereto. As another example, the material of the external catheter 100 may include at least one of silicone, latex, polyurethane, polyisoprene, and PVC.

The external catheter 100 may include a coupling part 120, a stretchable part 130, and a guide part 140. Here, the guide part 140 may be located on one side of the external catheter 100, the coupling part 120 may be located on the other side of the external catheter 100, and the stretchable part 130 may be located between the one side and the other side of the external catheter 100. For example, one side of the external catheter 100 may be defined as the front of the external catheter 100, and the other side of the external catheter 100 may be defined as the back of the external catheter 100.

The coupling part 120 may be coupled to the outer circumference of the internal catheter 300. As an example, the coupling part 120 may have a ring shape.

The stretchable part 130 is connected to one side of the connecting part 200 and may be stretchable in the direction of movement of the internal catheter 300. For example, the stretchable part 130 may have a bellows shape. Here, when the stretchable part 130 is extended, the overall length of the external catheter 100 may be lengthened, and when the stretchable part 130 is retracted, the overall length of the external catheter 100 may be shortened.

The guide part 140 is connected to one side of the stretchable part 130 and may guide the movement of the internal catheter 300. For example, the guide part 140 may have a ring shape. Meanwhile, the balloon 200 may be connected to one side of the guide part 140.

The balloon 200 is connected to one side of the external catheter 100, may be inflated or deflated, and may be inserted into or expelled from a target site of a living body. This balloon 200 may be inflated while inserted into the target site of the living body and fixed to a lesion tissue and the target site of the living body. For example, the balloon 200 may be inflated by fluid injection. For reference, the target site of the living body may be an internal space of the living body that may be inserted without an incision in the living body (e.g., one of the oral cavity, nasal cavity, pharynx, larynx, esophagus, stomach, duodenum, large intestine, and rectum), an internal space of the living body that may be inserted through an incision (e.g., one of the thoracic cavity, abdominal cavity, and inside the skin), but the present disclosure is not limited thereto, and may be applied to other tissues of the living body. In addition, the lesion tissue may be cancer tissue.

For example, the material of the balloon 200 may include at least one of silicone, latex, polyurethane, and polyisoprene.

The internal catheter 300 may be installed to be movable in a first direction, inserted into the balloon 200, or in a second direction, expelled from the balloon 200 along the external catheter 100. Here, the first direction movement may be defined as forward movement, and the second direction movement may be defined as backward movement. In addition, the internal catheter 300 may be expelled from the balloon 200 while the balloon 200 is inflated and fixed to the target area of the living body.

For example, when the internal catheter 300 is moved in the first direction inserted into the balloon 200, one side of the internal catheter 300 may be inserted into the inside of the balloon 200. At this time, the one side of the internal catheter 300 inserted into the inside of the balloon 200 may provide rigidity to the balloon 200 when the balloon 200 is inserted into the target site of the living body, thereby overcoming the insertion resistance applied to the balloon 200 in the living body. Thereafter, the balloon 200 inserted into the target site of the living body may be inflated and fixed to the lesion tissue and target site of the living body. Subsequently, one side of the internal catheter 300 inserted into the inside of the balloon 200 may be discharged from the inside of the balloon 200 by the driving part.

As an example, a valve 310 for sealing the internal catheter may be installed on the other side of the internal catheter 300. This valve 310 may serve to allow the inflow of the fluid supplied from a fluid supply part and prevent the outflow of the corresponding fluid.

As an example, the valve 310 may be composed of at least one of a check valve, a stopcock, and a clamp.

For example, the external catheter 100 or the internal catheter 300 may be fixed around the target site of the living body so that the position of the balloon 200 in the target site of the living body is maintained.

The driving part may move the internal catheter 300 inserted inside the balloon 200 in the second direction and discharge the internal catheter 300 while the balloon 200 inserted in the target site of the living body is inflated and fixed. This driving part may be operated under the control of a processor.

The driving part may include an actuator and the fluid supply part.

The actuator may move the internal catheter 300 in the second direction. For example, the actuator may move the internal catheter 300 in the second direction while the balloon 200 inserted in the target site of the living body is inflated and fixed under the control of the processor.

The fluid supply part may inject fluid into the balloon 200 so that the internal catheter 300 moves in the second direction by pressure of the fluid injected into the balloon 200. Here, the pressure of the fluid injected into the balloon 200 may provide part or all of the force required to move the internal catheter 300 in the second direction. For example, in the case that the pressure of the fluid injected into the balloon 200 provides only part of the force required to move the internal catheter 300 in the second direction, the pressure of the fluid injected into the balloon 200 may serve to assist the force when the user pulls the internal catheter 300 in the second direction. In addition, in the case that the pressure of the fluid injected into the balloon 200 provides all of the force required to move the internal catheter 300 in the second direction, the pressure of the fluid injected into the balloon 200 itself may serve as a driving force to move the internal catheter 300 in the second direction.

Meanwhile, the diameter and material of the balloon 200 may be initially set so that the pressure required for initial inflation is greater than the pressure required for the second direction movement of the internal catheter 300. Accordingly, when the fluid supply part injects fluid into the interior of the balloon 200, by the gas filled inside the balloon 200, the internal catheter 300 may be pushed to move in the second direction.

The fluid supply part may inject fluid through the internal catheter 300 or the external catheter 100.

Hereinafter, a process of inserting and fixing a balloon catheter according to one embodiment of the present disclosure into the target site of the living body will be described.

First, the internal catheter 300 penetrated into the external catheter 100 is moved in the first direction and inserted into the inside of a balloon 200.

Subsequently, the balloon 200 is inserted into the target site of the living body. At this time, one side of the internal catheter 300 inserted into the inside of the balloon 200 may provide rigidity to the balloon 200 when the balloon 200 is inserted into the target site of the living body, thereby overcoming the insertion resistance applied to the balloon 200 inside the living body.

Next, the balloon 200 is inflated and fixed to the lesion tissue and target site of the living body. At this time, the balloon 200 may be inflated by fluid injection. For example, the fluid injection of the balloon 200 may be performed through the fluid supply part of the driving part.

Thereafter, the driving part moves the internal catheter 300 in the second direction so that one side of the internal catheter 300 is discharged from the inside of the balloon 200.

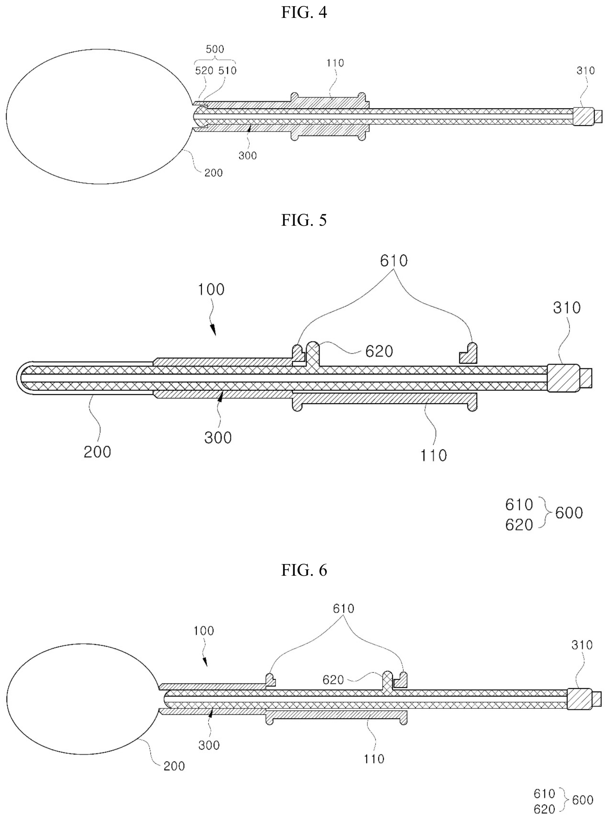

FIG. 3 is a cross-sectional view illustrating a first stopper of a balloon catheter according to an embodiment of the present disclosure, and FIG. 4 is a cross-sectional view illustrating the balloon of FIG. 3 in an inflated state.

As shown in FIG. 3, the balloon catheter according to an embodiment of the present disclosure may further include a first stopper 500, unlike the example of FIG. 1. In this example, the inner circumference of the external catheter 100 may be closely attached to the outer circumference of the internal catheter 300. Therefore, air leakage or fluid leakage between the external catheter 100 and the internal catheter 300 may be prevented.

The first stopper 500 may serve to limit the movement range of the internal catheter 300 in the second direction to prevent the internal catheter 300 from dislodging from the external catheter 100. The first stopper 500 may include a hook groove 510 and a hook 520.

The hook groove 510 may be formed on one side of the external catheter 100. For example, the hook groove 510 may be formed by being sunken along the inner circumference of one side of the external catheter 100. At this time, the hook groove 510 may have a ring shape.

The hook 520 is formed in the internal catheter 300 and may be caught in the hook groove 510 when the internal catheter 300 moves in the second direction. For example, the hook 520 may have a shape corresponding to the hook groove 510.

In this example, when the internal catheter 300 moves in the second direction, the hook 520 is caught in the hook groove 510 and the range of movement of the internal catheter 300 in the second direction may be limited (Refer to FIG. 4).

FIG. 5 is a cross-sectional view illustrating a second stopper of a balloon catheter according to one embodiment of the present disclosure, and FIG. 6 is a cross-sectional view illustrating the balloon of FIG. 5 in an inflated state.

As illustrated in FIG. 5, the balloon catheter according to one embodiment of the present disclosure may further include a second stopper 600, unlike the example of FIG. 1. In this example, the inner circumference of the external catheter 100 may be closely attached to the outer circumference of the internal catheter 300. Therefore, air leakage or fluid leakage through the space between the external catheter 100 and the internal catheter 300 may be prevented.

The second stopper 600 may limit the first direction movement range and the second direction movement range of the internal catheter 300 to prevent the internal catheter 300 from dislodging from the external catheter 100. This second stopper 600 may include two catch protrusions 610 and a moving protrusion 620.

The two catch protrusions 610 may be coupled to the outer circumference of the external catheter 100 with a gap along the direction of movement of the internal catheter 300. For example, the two catch protrusions 610 may have a shape that protrudes vertically from the outer circumference of the external catheter 100.

The moving protrusion 620 may be coupled to the outer circumference of the internal catheter 300 and may be positioned between the two studs 610. For example, the moving protrusion 620 may have a shape that protrudes vertically from the outer circumference of the internal catheter 300.

In this example, when the internal catheter 300 moves in the first direction, the moving protrusion 620 may be caught by the one located closer to one side of the external catheter 100 among the two catch protrusions 610, and the movement range of the internal catheter 300 in the first direction may be limited (Refer to FIG. 6).

In addition, when the internal catheter 300 moves in the second direction, the moving protrusion 620 may be caught by the one located closer to the other side of the external catheter 100 among the two catch protrusions 610, and the movement range of the internal catheter 300 in the second direction may be limited (Refer to FIG. 6).

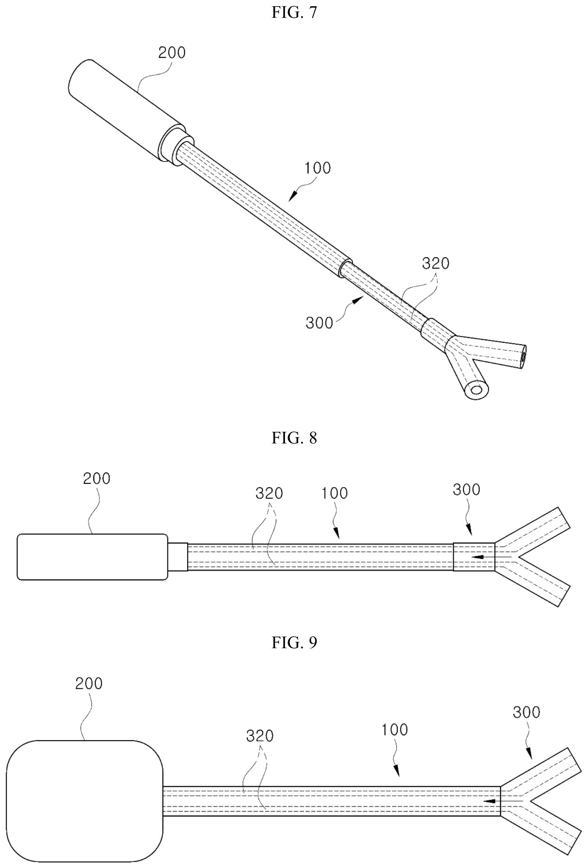

FIGS. 7 to 10 are cross-sectional views illustrating an operation process of a balloon catheter according to another embodiment of the present disclosure.

As shown in FIG. 7, the balloon catheter according to another embodiment of the present disclosure may have the internal catheter 300 with multiple lumens 320, unlike the example of FIG. 1, and may include a third stopper 700.

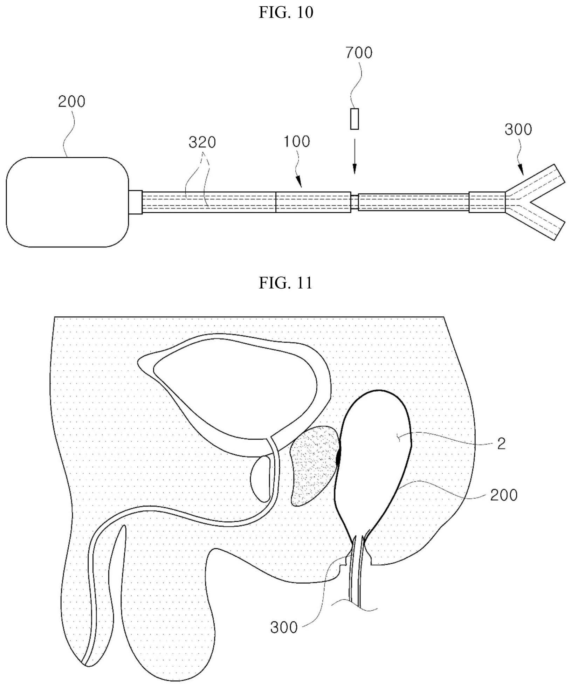

The third stopper 700 may be detachably fixed to the internal catheter 300 and may serve to limit the movement range of the internal catheter 300 in the first direction (Refer to FIG. 10). As an example, the third stopper 700 may have a U-shape. This third stopper 700 is fixed to the outer circumference of the internal catheter 300 in contact with the other side of the external catheter 100 while one side of the internal catheter 300 is discharged from the inside of the balloon 200, thereby limiting the range of movement of the internal catheter 300 in the first direction.

Hereinafter, a process of inserting and fixing the balloon catheter according to another embodiment of the present disclosure into a target site of a living body will be described.

First, the internal catheter 300 moves along the external catheter in the first direction and inserted into the inside of the balloon 200 (Refer to FIG. 8).

Next, the balloon 200 is inserted into the target site of the living body. At this time, one side of the internal catheter 300 inserted into the inside of the balloon 200 may provide rigidity to the balloon 200 when the balloon 200 is inserted into the target site of the living body, thereby overcoming the insertion resistance applied to the balloon 200 inside the living body.

Thereafter, the balloon 200 is inflated and fixed to the lesion tissue and target site of the living body. At this time, the balloon 200 may be inflated by fluid injection. For example, the fluid injection of the balloon 200 may be performed through the fluid supply part of the driving part (Refer to FIG. 9).

Next, the driving part moves the internal catheter 300 in the second direction so that one side of the internal catheter 300 is discharged from the inside of the balloon 200. Thereafter, the third stopper 700 is fixed to the outer circumference of the internal catheter 300 that contacts the other side of the external catheter 100 while one side of the internal catheter 300 is discharged from the inside of the balloon 200, thereby limiting the movement range of the internal catheter 300 in the first direction (Refer to FIG. 10).

As an example, during radiation therapy, one of X-rays, electrons, protons, and carbon particles of 1 MeV or more may be used.

For reference, in the case that there is a balloon with a large or small density inside the living body compared to the target area of the living body, it may take more time to calculate the radiation dose for the radiation treatment plan. Therefore, the balloon 200 may be set to a predetermined shape and density to shorten the radiation dose calculation time. At this time, the shape of the balloon 200 may be set to one of a sphere, a hemisphere, an ellipsoid, a cylinder, and a square cylinder. Furthermore, the basic shape of the balloon 200 may be adjusted when it is ejected, and the expansion shape may be adjusted by adjusting the thickness of each unit area of the balloon 200. In addition, the internal density of the balloon 200 may be adjusted by adjusting the composition of the material injected into the balloon 200.

For example, a sensing module for measuring the volume and pressure of the balloon 200 may be installed in the external catheter 100 or the internal catheter 300, and the sensing module may provide the volume and pressure data of the balloon 200 to the operator for monitoring the volume and pressure of the balloon 200.

For example, the internal temperature of the balloon 200 may be controlled through a fluid circulation device that circulates the fluid injected into two or more lumens 320 of the internal catheter 300. At this time, the temperature of the balloon 200 may affect the therapeutic effect or side effect of the target area of the living body that comes into contact with the balloon 200, and the higher the temperature of the balloon 200, the greater the effect and side effect due to radiation, and the lower the temperature of the balloon 200, the less the effect and side effect due to radiation.

For example, the balloon 200 may be coated with a radiation-sensitive material that changes color when exposed to radiation. Accordingly, when the balloon 200 is exposed to radiation, the color of the balloon 200 may change, and through this, the radiation dose irradiated and absorbed by the balloon 200 may be calculated, and through this, the radiation dose irradiated and absorbed by the target area of the living body in contact with the balloon 200 may be calculated. Meanwhile, the color change of the balloon 200 may be identified in real time through an endoscope inserted into the target area of the living body, or may be identified through the balloon 200 discharged from the target area of the living body after the end of radiation treatment.

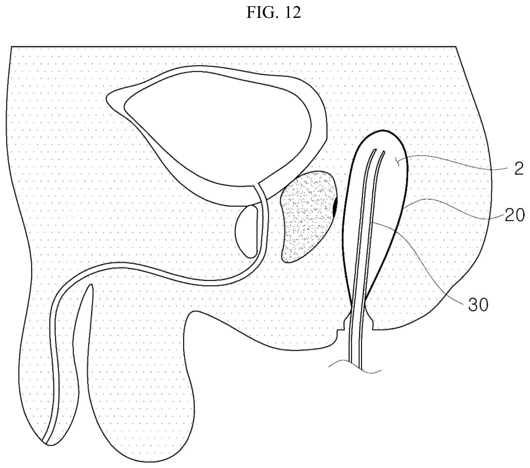

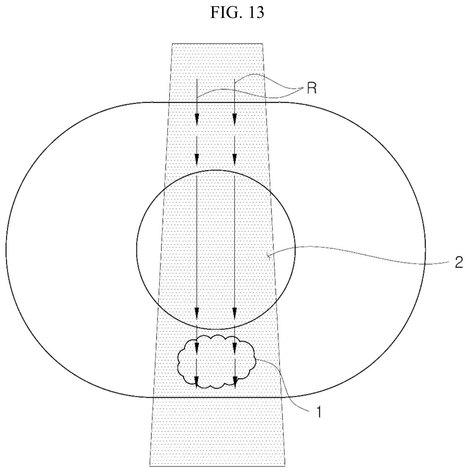

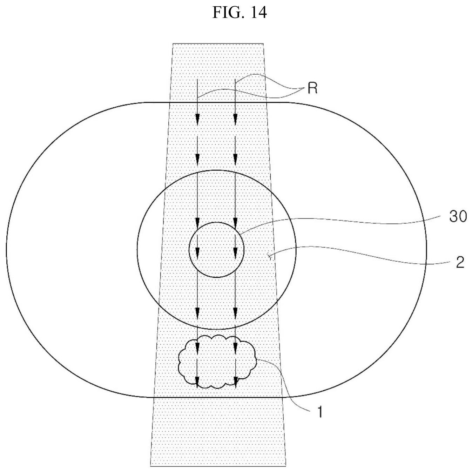

FIG. 11 is a schematic diagram illustrating a state in which a balloon of a balloon catheter according to one embodiment of the present disclosure is fixed to a target site of a living body, FIG. 12 is a schematic diagram illustrating a state in which a balloon of a conventional balloon catheter is fixed to a target site of a living body, FIG. 13 is a cross-sectional diagram illustrating a process of radiation therapy of a target site of a living body using a balloon catheter according to one embodiment of the present disclosure, and FIG. 14 is a cross-sectional diagram illustrating a process of radiation therapy of a target site of a living body using a conventional balloon catheter.

Referring to FIG. 11, in the state that the balloon 200 of the balloon catheter according to one embodiment of the present disclosure is fixed to a target site 2 of the living body, the internal catheter 300 does not exist inside the balloon 200 fixed to the target site 2 of the living body. Therefore, during radiation therapy, it may be identified that the radiation R penetrating the inside of the balloon 200 reaches the lesion tissue 1 of the living body without being affected by the internal catheter 300 (Refer to FIG. 13).

On the other hand, referring to FIG.12, in the state that the balloon 20 of the conventional balloon catheter is fixed to the target area of the living body, it may be identified that the internal catheter 30 exists inside the balloon 20 fixed to the target area 2 of the living body. Therefore, during radiation therapy, it may be identified that the radiation R penetrating the inside of the balloon 200 is affected by the internal catheter 300 and reaches the lesion tissue 1 of the living body (Refer to FIG. 14). Due to this, the accuracy of radiation therapy in the conventional technology may be reduced.

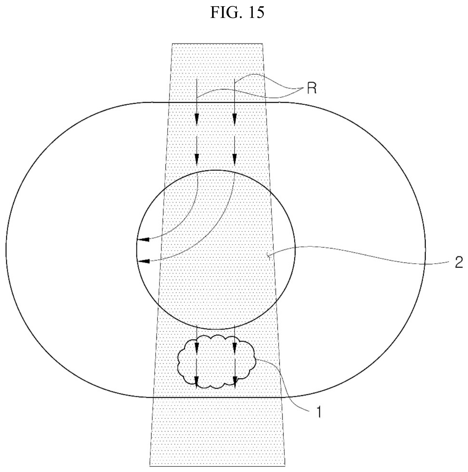

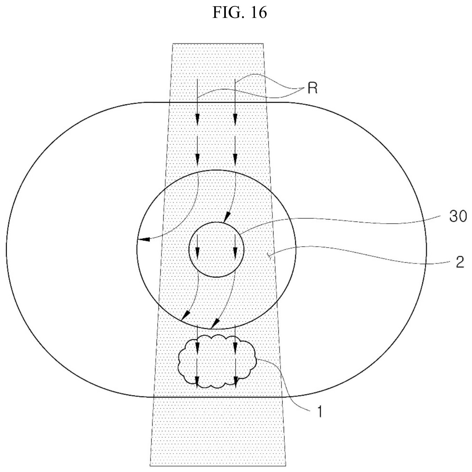

FIG. 15 is a cross-sectional view illustrating an MCRT treatment process of a target area of a living body using a balloon catheter according to one embodiment of the present disclosure, and FIG. 16 is a cross-sectional view illustrating an MCRT treatment process of a target area of a living body using a balloon catheter according to one embodiment of the present disclosure.

Referring to FIG. 15, in the balloon catheter according to one embodiment of the present disclosure, there is no internal catheter 300 inside the balloon 200 fixed to the target area 2 of the living body. Accordingly, in MCRT (Magnetic Controlled Radiation Therapy) that controls the path of radiation using a magnetic field, since there is no internal catheter 300 on the path of radiation penetrating the inside of the balloon 200, the generation of secondary electrons due to the influence of the internal catheter 300 on the radiation may be prevented.

On the other hand, the conventional balloon catheter has an internal catheter 300 inside the balloon 200 fixed to the target site 2 of the living body. Therefore, during MCRT, which controls the path of radiation using a magnetic field, since the internal catheter 30 is on the path of radiation penetrating the inside of the balloon 20, secondary electrons are generated due to the influence of the internal catheter 30 on the radiation. Due to this, the conventional technology has a reduced accuracy in controlling the path of radiation by MCRT.

Therefore, the balloon catheter according to one embodiment of the present disclosure has the effect of improving the accuracy of radiation treatment for the lesion tissue and target site of the living body during radiation treatment by enabling the discharge of the internal catheter inserted inside the balloon while the balloon is inflated and fixing the lesion tissue and target site of the living body.

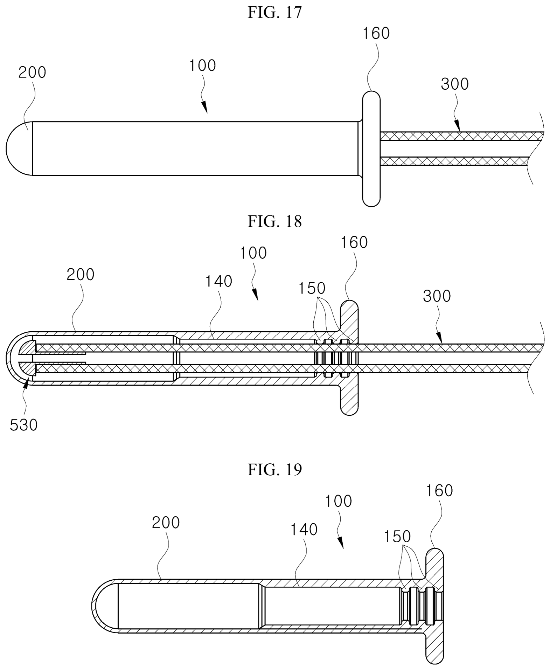

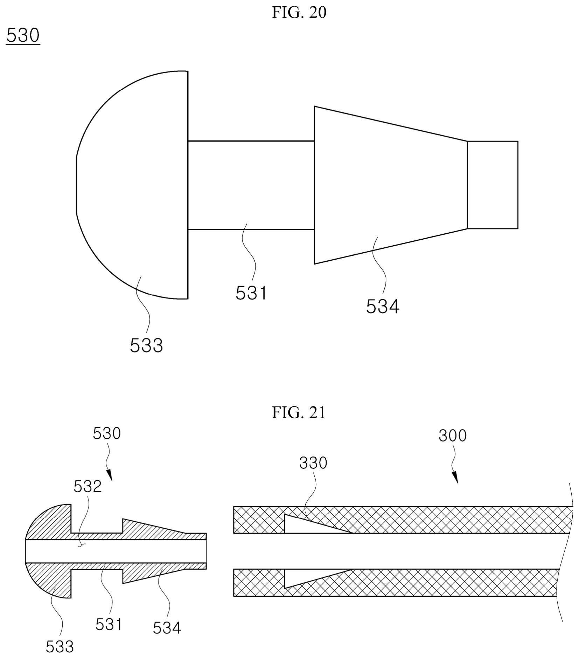

FIG. 17 is a perspective view of a balloon catheter according to still another embodiment of the present disclosure, including a plug and wing part, FIG. 18 is a cross-sectional view of a balloon catheter according to still another embodiment of the present disclosure, including a plug and wing part, FIG. 19 is a cross-sectional view showing an external catheter of a balloon catheter according to still another embodiment of the present disclosure, and FIG. 20 is a perspective view showing a plug of a balloon catheter according to still another embodiment of the present disclosure.

Referring to FIGS. 17 to 20, unlike the example of FIG. 1, a balloon catheter according to still another embodiment of the present disclosure may include the external catheter 100 including the guide part 140, a sealing part 150, and a wing part 160.

The guide part 140 may be the main body of the external catheter 100. The internal catheter 300 may be installed within the guide part 140 to be movable in the first or second direction. At this time, the guide part 140 may serve to guide the movement of the internal catheter 300.

The guide part 140 may be connected to the balloon 200 to guide the movement of the internal catheter 300. For example, the balloon 200 may be integrally connected to one side of the guide part 140. Meanwhile, when fluid is injected into the guide part 140 and the balloon 200, the guide part 140 and the balloon 200 may be configured as follows so that the guide part 140 is not substantially inflated by the fluid injection, and only the balloon 200 is selectively inflated.

For example, the balloon 200 may have a smaller thickness than the guide part 140.

For another example, the balloon 200 may have a smaller inner diameter than the guide part 140.

For another example, the balloon 200 may be formed of a material having a higher elastic modulus than the guide part 140.

The sealing part 150 may protrude from the guide part 140 toward the internal catheter 300 and be in close contact with the internal catheter 300. The sealing part 150 may serve to seal the interior of the guide part 140.

For example, the sealing part 150 may be formed as a single piece.

As another example, the sealing part 150 may be configured in multiple pieces. Here, the multiple sealing parts 150 may be formed at an interval along the direction of movement of the internal catheter 300. The multiple sealing parts 150 may serve to further seal the interior of the guide part 140.

The wing part 160 may be formed to protrude from the external catheter 100 so as to limit the depth at which the external catheter 100 is inserted into the target site of the living body. Therefore, the wing part 160 may prevent the external catheter 100 from being excessively inserted into the target site of the living body.

In the balloon catheter according to another embodiment of the present disclosure, unlike the example of FIG. 1, the first stopper 500 may further include a plug 530.

The plug 530 may be connected to one side of the internal catheter 300 and may serve to limit the range of movement of the internal catheter 300 in the second direction to prevent the internal catheter 300 from being detached from the external catheter 100.

The plug 530 may include a plug body 531, a channel 532, a step 533, and a catch protrusion 534.

The plug body 531 may be connected to one side of the internal catheter 300. The channel 532 may be formed in the plug body 531.

The channel 532 may be formed in the plug body 531 and communicate with the internal catheter 300. For example, the channel 532 may be formed along the central axis of the plug body 531.

The step 533 may protrude from one side of the plug body 531 and may be caught by the sealing part 150 as the internal catheter 300 moves in the second direction. Therefore, the step 533 may prevent the internal catheter 300 from being detached from the external catheter 100.

For example, the step 533 may have a shape in which the cross-sectional area increases as it moves in the second direction. In this case, the step 533 may have a cone shape.

The catch protrusion 534 may protrude from the other side of the plug body 531 and may be caught and fixed around the inner circumference of the internal catheter 300.

FIG. 21 is an exploded cross-sectional view illustrating a plug and an internal catheter of a balloon catheter according to still another embodiment of the present disclosure.

As illustrated in FIG. 21, the catch protrusion 534 may have a shape whose cross-sectional area decreases in the second direction. Here, an engaging groove 330 may be formed on the inner circumference of the internal catheter 300 to engage and secure the catch protrusion 534. Accordingly, when the plug 530 is inserted into the internal catheter 300, the catch protrusion 534 is engaged and secured in the engaging groove 330, thereby allowing the plug 530 to be detachably secured to the internal catheter 330.

For example, the engaging groove 330 may have a shape corresponding to the catch protrusion 534.

Hereinafter, the process of inserting and securing a balloon catheter according to another embodiment of the present disclosure into a target site of a living body will be described.

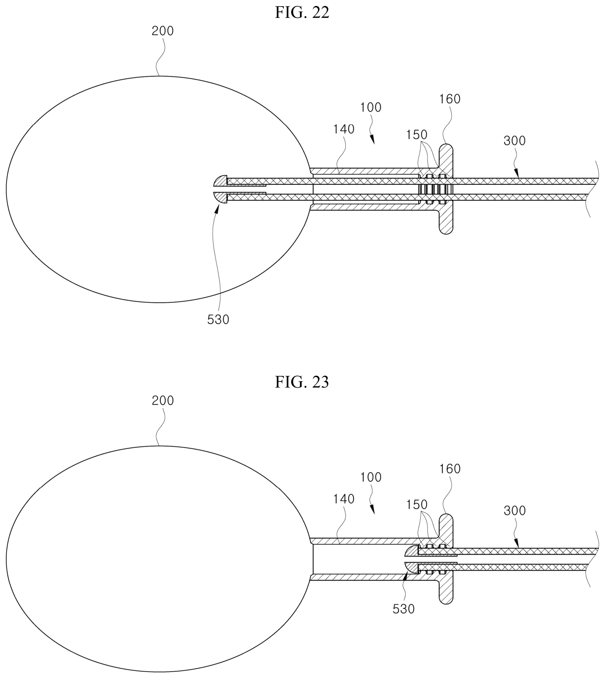

FIGS. 22 and 23 are cross-sectional views illustrating the operation of a balloon catheter according to still another embodiment of the present disclosure.

First, the internal catheter 300 is moved in the first direction along the guide part 140 of the external catheter 100 and inserted into the interior of the balloon 200. In this case, the sealing part 150 of the external catheter 100 may serve to seal the interior of the balloon 200 and the interior of the guide part 140.

Next, the balloon 200 is inserted into the target site of the living body. In this case, one side of the internal catheter 300 inserted into the balloon 200 may provide rigidity to the balloon 200 when it is inserted into the target site of the living body, thereby overcoming the insertion resistance applied to the balloon 200 within the living body (Refer to FIG. 22).

Next, the balloon 200 is inflated and secured to the lesion tissue and target site of the living body. In this case, the balloon 200 may be inflated by fluid injection. For example, the fluid injection into the balloon 200 may be performed through the fluid supply part of the driving part.

Thereafter, the driving part moves the internal catheter 300 in the second direction so that one side of the internal catheter 300 is discharged from the interior of the balloon 200.

In this case, as the internal catheter 300 moves in the second direction, the step 533 of the plug 530 fixed to the internal catheter 300 is caught on the sealing part 150 of the external catheter 100, thereby preventing the internal catheter 300 from being detached from the external catheter 100 (Refer to FIG. 23).

Although the embodiments of the present disclosure have been described above with reference to the attached drawings, those skilled in the art will understand that the present disclosure may be implemented in other specific forms without changing the technical idea or essential features thereof. Therefore, it should be understood that the embodiments described above are exemplary in all respects and not restrictive.

## Claims

1. A balloon catheter comprising:
an external catheter;
a balloon connected to one end of the external catheter, inflatable and deflatable, and configured to be inserted into or expelled from a target site of a living body; and
an internal catheter movable along the external catheter in a first direction for insertion into the balloon or a second direction for expelling the balloon,
wherein the internal catheter is expelled from the balloon while the balloon is inflated and fixed to the target site of the living body.

2. The balloon catheter of claim 1, further comprising:
a first stopper configured to limit a movement range of the internal catheter in the second direction to prevent the internal catheter from being dislodged from the external catheter.

3. The balloon catheter of claim 2, wherein the first stopper comprises:
a hook groove formed in the external catheter; and
a hook formed in the internal catheter and hooked to the hook groove as the internal catheter moves in the second direction.

4. The balloon catheter of claim 2,
wherein the first stopper includes a plug connected to one side of the internal catheter, and
wherein the plug includes:
a plug body connected to one side of the internal catheter;
a channel formed in the plug body and communicating with the internal catheter; and
a step protruding from one side of the plug body and engaging a sealing part of the external catheter as the internal catheter moves in the second direction.

5. The balloon catheter of claim 4,
wherein the plug further includes a catch protrusion from the other side of the plug body and catching and fixed on an inner circumference of the internal catheter.

6. The balloon catheter of claim 1, further comprising:
a second stopper configured to limit a movement range of the internal catheter in the first direction and a movement range of the second direction to prevent the internal catheter from dislodging from the external catheter.

7. The balloon catheter of claim 6, wherein the second stopper comprises:
two catch protrusions spaced apart with a spacing and connected along a movement direction of the internal catheter to the outer circumference of the external catheter; and
a moving protrusion connected to the outer circumference of the internal catheter and is arranged between the two catch protrusions.

8. The balloon catheter of claim 1, further comprising:
a driving part that moves the internal catheter in the second direction while the balloon is inflated and fixed to the target site of the living body.

9. The balloon catheter of claim 8, wherein the driving part comprises:
an actuator configured to move the internal catheter in the second direction.

10. The balloon catheter of claim 8, wherein the driving part comprises:
a fluid supply part configured to inject fluid into the balloon so that pressure from the fluid injected into the balloon assists the movement of the internal catheter in the second direction.

11. The balloon catheter of claim 1,
wherein the external catheter includes:
a guide part connected to the ballon to guide the movement of the internal catheter; and
a sealing part protruding from the guide part toward the internal catheter and in close contact with the internal catheter.

12. The balloon catheter of claim 1, further comprising a wing part protruding from an outer periphery of the external catheter to limit a depth at which the external catheter is inserted into the target site of the living body.
